# EUROPEAN PATENT APPLICATION

(11) **EP 1 690 493 A1**
(43) Date of publication of application: **16.08.2006**
(21) Application number: 06002267.0
(22) Date of filing: 03.02.2006
(51) Int. Cl.: A61B 5/022

(54) **Blood-pressure monitor**

(30) Priority: 14.02.2005 JP 2005036771
(71) Applicant: TANITA CORPORATION, Tokyo (JP)
(72) Inventor: Iwasawa, Toshinobu, Tokyo (JP)
(74) Representative: Grünecker, Kinkeldey, Stockmair & Schwanhäusser Anwaltssozietät

(57) **Abstract**

Disclosed is a blood-pressure monitor, which comprises signal generation means for generating a breathing-guidance signal during a blood-pressure measurement, and display means for displaying an indication for guiding breathing based on the breathing-guidance signal. The blood-pressure monitor of the present invention can display an indication for guiding resting breathing during a blood-pressure measurement so as to perform a stable blood-pressure measurement.

## Description

### TECHNICAL FIELD

The present invention relates to a blood-pressure monitor capable of performing stable blood-pressure measurement.

### BACKGROUND ART

Generally, a blood-pressure value varies depending on time of day, measurement date and body conditions, such as exercise or mental tension. Therefore, in medical institutions, it has been recommended to steady patient's breathing before blood-pressure measurement. Further, a method of correcting maximal and minimal blood pressures using pulse amplitudes has been proposed [see, for example, Japanese Patent Laid-Open Publication No. 11-33005 (Patent Publication 1)].

In order to measure a stable blood-pressure value, there has also been known a technique of performing a plurality of blood-pressure measurements and calculating an average value of the measurement results.

However, even if a patient steadies his/her breathing before measurement, the patient is apt to forget to maintain a constant breathing due to preoccupation with changes in blood-pressure value or surrounding during the measurement. Further, even if the patient tries to steady his/her breathing, an interval between breaths does not become constant due to body condition during the measurement to cause difficulty in performing stable blood-pressure measurement.

### DISCLOSURE OF THE INVENTION

In view of the above circumstances, it is an object of the present invention to provide a blood-pressure monitor capable of reliably performing a stable blood-pressure measurement.

In order to achieve the above object, the present invention provide a blood-pressure monitor comprising signal generation means for generating a breathing-guidance signal during a blood-pressure measurement, and display means for displaying an indication for guiding breathing based on the breathing-guidance signal.

In the blood-pressure monitor of the present invention, the breathing-guidance signal may comprise an exhalation signal and an inhalation signal.

Further, the display means may display inhalation and exhalation by way of a change in the intensity of a bar code. Alternatively, the display means may display inhalation and exhalation by way of a change in the size of a graphic displayed. The display means may display inhalation and exhalation by way of a change in the displayed status of a graphic modeled after respiratory organs.

In the blood-pressure monitor of the present invention, the signal generation means may be operable to continuously generate the breathing-guidance signal during the blood-pressure measurement.

According to the blood-pressure monitor of the present invention, during a blood-pressure measurement, the display means can display an indication for guiding breathing based on the breathing-guidance signal generated by the signal generation means. This makes it possible to allow the subject to breathe according to the indication on the display means so as to perform a stable blood-pressure measurement.

In the blood-pressure monitor of the present invention, the breathing-guidance signal may comprise an exhalation signal and an inhalation signal. This makes it possible to facilitate the control of displaying an indication for guiding breathing.

Further, the display means may display inhalation and exhalation by way of a change in the intensity of a bar code. Alternatively, the display means may display inhalation and exhalation by way of a change in the size of a graphic displayed. This makes it possible to visually guide breathing. The display means may display inhalation and exhalation by way of a change in the displayed status of a graphic modeled after respiratory organs. This makes it possible to visually guide breathing.

In the blood-pressure monitor of the present invention, the signal generation means may be operable to continuously generate the breathing-guidance signal during the blood-pressure measurement. This makes it possible to stably perform the entire blood-pressure measurement without influence from surrounding.

### BRIEF DESCRIPTION OF DRAWINGS

FIG. 1 is a block diagram showing a blood-pressure monitor according to one embodiment of the present invention.
FIG. 2 is a flowchart showing an operation of the blood-pressure monitor according to the embodiment.
FIG. 3 is a flowchart showing a process of guiding breathing in the blood-pressure monitor according to the embodiment.
FIG. 4 is a schematic diagram showing a display section of a main unit of the blood-pressure monitor according to the embodiment.
FIG. 5 is a schematic diagram showing another example of a breathing-guidance indication in the blood-pressure monitor according to the embodiment.
FIG. 6 is a schematic diagram showing yet another example of a breathing-guidance indication in the blood-pressure monitor according to the embodiment.

### BEST MODE FOR CARRYING OUT THE INVENTION

A blood-pressure monitor of the present invention comprises signal generation means for generating a breathing-guidance signal during a blood-pressure measurement, and display means for displaying an indication for guiding breathing based on the breathing-guidance signal.

In the blood-pressure monitor of the present invention, the breathing-guidance signal may comprise an exhalation signal and an inhalation signal.

Further, the display means may display inhalation and exhalation by way of a change in the intensity of a bar code. Alternatively, the display means may display inhalation and exhalation by way of a change in the size of a graphic displayed. The display means may display inhalation and exhalation by way of a change in the displayed status of a graphic modeled after respiratory organs.

In the blood-pressure monitor of the present invention, the signal generation means may be operable to continuously generate the breathing-guidance signal during the blood-pressure measurement.

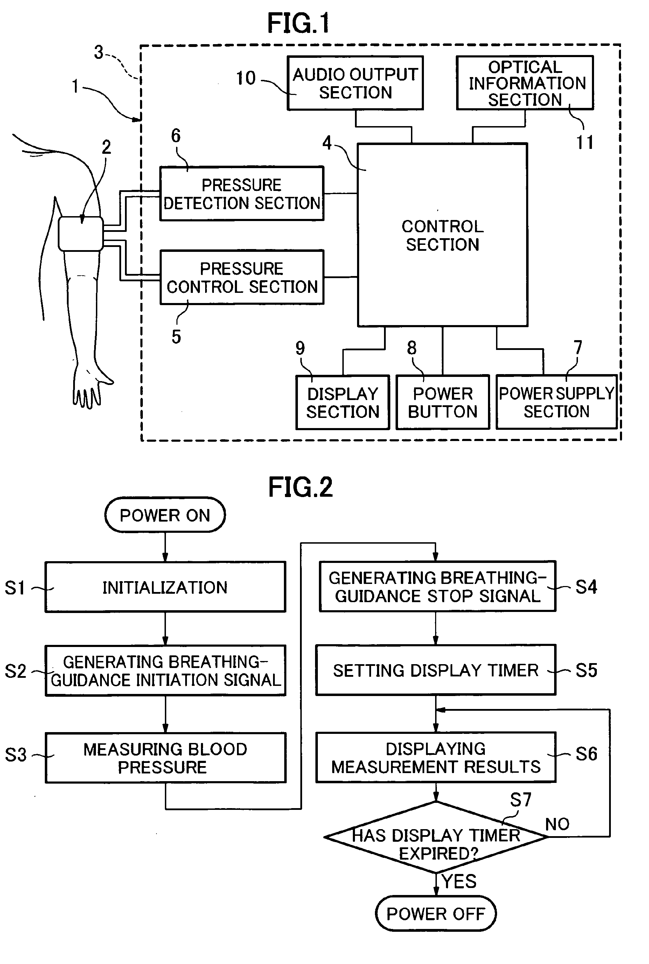

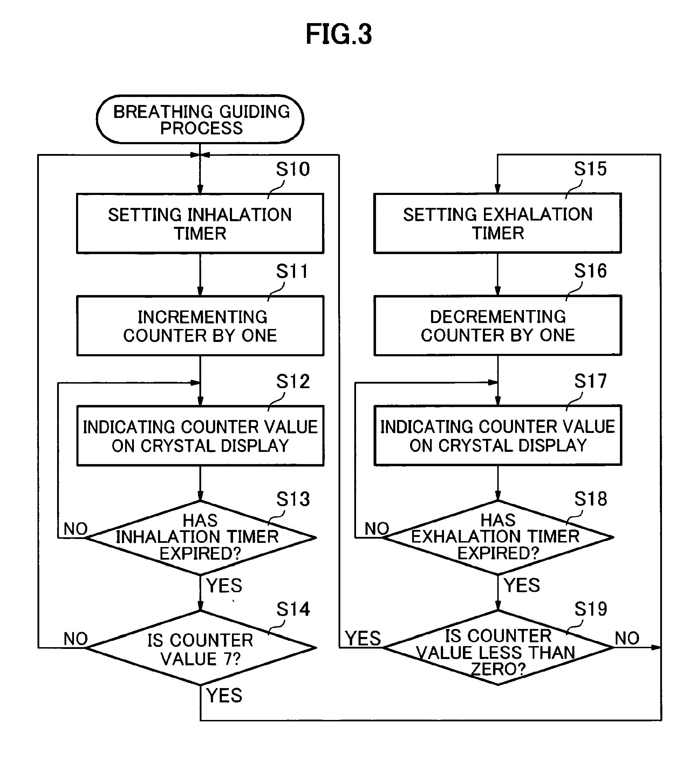

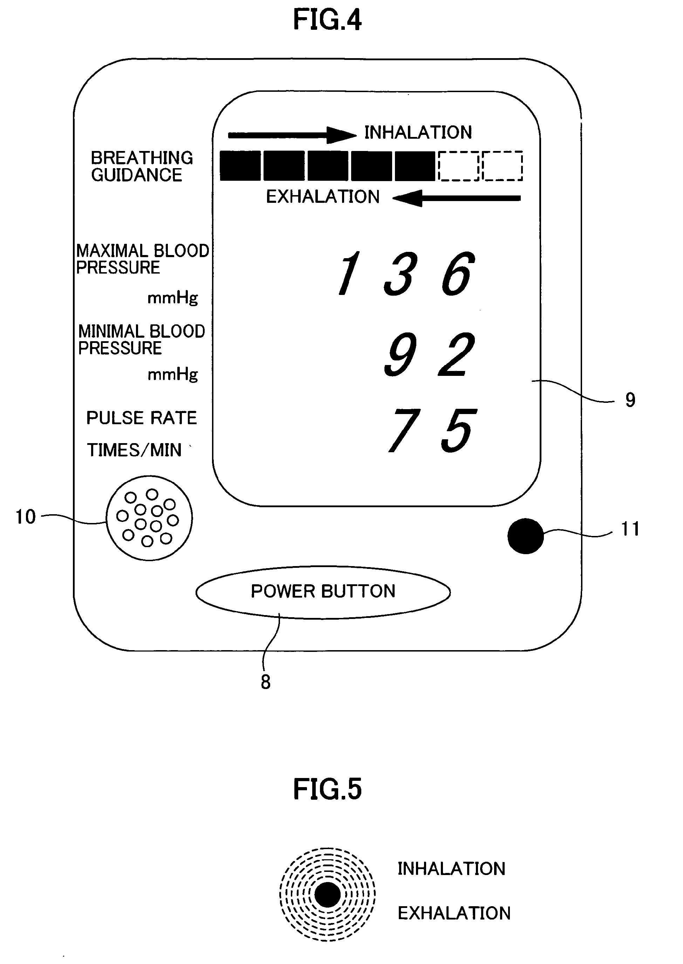

With reference to FIGS. 1 to 4, a blood-pressure monitor according to one embodiment of the present invention will now be described. FIG. 1 is a block diagram showing a relationship between each section of the blood-pressure monitor and the upper arm of a subject. FIGS. 2 and 3 are schematic control flowcharts of the blood-pressure monitor. FIG. 4 is a schematic diagram showing a display of the blood-pressure monitor, wherein a power button 8 is disposed on the lower side of a display section 9 of a main unit 3.

As shown in FIG. 1, the blood-pressure monitor 1 comprises a cuff 2 adapted to be wound around the upper arm of a subject, and the main unit 3. The main unit 3 includes a control section 4 internally provided with a microcomputer and adapted to generally control the blood-pressure monitor 1, a pressure control section 5 electrically connected to the control section 4 and adapted to selectively increase and reduce a pressure of the cuff 2, a pressure detection section 6 electrically connected to the control section 4 and adapted to detect a pressure of the cuff 2, a power supply section 7 for supplying an electric power to the main unit 3, the power button 8 for selectively turning on and off the supply of the electric power to the main unit 3, and the display section 9 for displaying a measurement result. These components are the same as those in a conventional widely-used blood-pressure monitor, and their detailed descriptions will be omitted.

As shown in FIG. 1, the blood-pressure monitor 1 further includes an audio output section 10 for informing the subject about a measurement result and a breathing guidance in an audio manner, and an optical information section 11 for informing the subject about a breathing guidance in an optical manner. Each of the audio output section 10 and the optical information section 11 is electrically connected to the control section 4.

With reference to FIGS. 2 to 4, an operation of the blood-pressure monitor 1 in FIG. 1 will be described below.

In FIG. 2, when the power button 8 is pressed, an electric power is supplied from the power supply section 7 to the entire main unit 3, and the microcomputer of the control section 4 initializes a timer, a counter and others therein in Step S1. Then, in Step S2, the microcomputer generates a breathing-guidance initiation signal. In response to the generation of the breathing-guidance initiation signal, the microcomputer additionally activates the flowchart of FIG. 3 in parallel with the flowchart of FIG. 2.

The flowchart of FIG. 3 is activated in Step S2 of FIG. 2, and will be operated in parallel with the flowchart of FIG. 2 until the microcomputer generates a breathing-guidance stop signal in Step S4 of FIG. 2. When the flowchart of FIG. 3 is activated, an inhalation signal is generated in Step S10, and an inhalation timer having a duration, for example, of 0.5 seconds, is set up. Then, in Step S11, a counter is set to "1". This counter is designed to be set to "zero" at the initialization, and subsequently set to either one of positive and negative values. In Step S12, the setup counter value is indicated on a plurality of barcode-like liquid-crystal-display blocks at the uppermost position in the display section 9. In this case, the counter value is "1", and therefore one of seven liquid-crystal-display blocks is turned on. Then, in Step S13, it is determined whether the timer has expired. If the timer has not expired, the state of the liquid-crystal-display brocks in Step S12 will be maintained. When it is determined that the timer has expired, the process advances to Step S14. In Step S14, it is determined whether the counter value is "7". In this embodiment, it is determined whether the counter value is "7" because the number of liquid-crystal-display blocks is seven. If the number of liquid-crystal-display blocks is ten, it will be determined whether the counter value is "10". That is, this determination is performed to check whether all of the liquid-crystal-display blocks are turned on.

When it is determined in Step S 14 that the counter value is not "7", the process returns to Step S10 to set the inhalation timer. Then, in Step S11, the counter is incremented by one, or the counter is set to "2". Thus, in Step S12, two of the seven liquid-crystal-display blocks are turned on. The above steps will be repeated until the counter value becomes "7". As above, this display is designed to turn on a plurality of barcode-like liquid-crystal-display blocks stepwise at given time intervals, specifically, to increase the number of liquid-crystal-display blocks to be turned on, from one up to seven at time intervals of 0.5 seconds, and provided as a means to prompt the subject to take in breath or inhale air.

When the counter value becomes "7" in Step S14, the process will advance to Step S15. In Step S15, an exhalation timer having a duration, for example, of 0.5 seconds, is set up. Then, in Step S16, the counter is decremented by one, or the counter is set to "6". Therefore, in Step S17, six of the seven liquid-crystal-display blocks are turned on. Then, in Step S18, it is determined whether the exhalation timer has expired. When it is determined that the exhalation timer has expired, the process advances to Step S 19. In Step S 19, it is determined whether the counter value is less than "zero". This determination is performed to check whether all of the barcode-like liquid-crystal-display blocks for an exhalation guidance are turned off. In this case, the determined in Step S19 is NO because the counter value is "6", and the process returns to Step S 15. In Step S16, the counter is decremented by one, or the counter is set to "5". Therefore, in Step S 17, five of the seven liquid-crystal-display blocks are turned on. The above steps will be repeated to reduce the number of liquid-crystal-display blocks to be turned on, one-by-one. This display is provided as a means to prompt the subject to expel his/her breath or exhale air. When the determination in Step S19 is YES or it is determined that all of the barcode-like liquid-crystal-display blocks for the exhalation guidance are turned off, the process returns to Step S10 to re-start the process for the inhalation guidance.

During the process of inhalation/exhalation guidance or breathing guidance, a blood pressure and a pulse rate are measured in Step S3 of FIG. 2. For example, the control section 4 drives the pressure control section 5 in such a manner as to increase a pressure of the cuff 2 up to a given value and then reduce the pressure, and simultaneously calculates maximal and minimal blood pressures based on a signal received from the pressure detection section 6. Further, before the pressure of the cuff 2 is increased to a high value, the control section 4 calculates a pulse rate based on a signal from the pressure detection section 6. The measurement of a blood pressure and a pulse rate is not limited to the above process, but may be performed by any other suitable conventional method.

When the measurement of a blood pressure and a pulse rate is completed, the microcomputer generates the breathing-guidance stop signal in Step S4 to stop the flowchart of FIG. 3. Then, in Step S5, a display timer having a duration, for example, of about 10 seconds, is set up. In Step S6, the maximal blood pressure, the minimal blood pressure and the pulse rate calculated in Step S6 are indicated on the display section 9. In an example illustrated in FIG. 4, the maximal blood pressure, the minimal blood pressure and the pulse rate are, respectively, 136 mm Hg, 92 mm Hg and 75. In Step S7, this indication will be continued until the display timer having the duration of about 10 seconds expires. When the duration expires, the power is automatically turned off to stop the operation of the blood-pressure monitor 1.

As mentioned above, the blood-pressure monitor of the present invention is designed to display the indication for guiding inhalation and exhalation of a subject and allow the subject to breathe according to the indication. This makes it possible to stabilize a blood pressure without influence from surrounding so as to accurately perform a blood-pressure measurement.

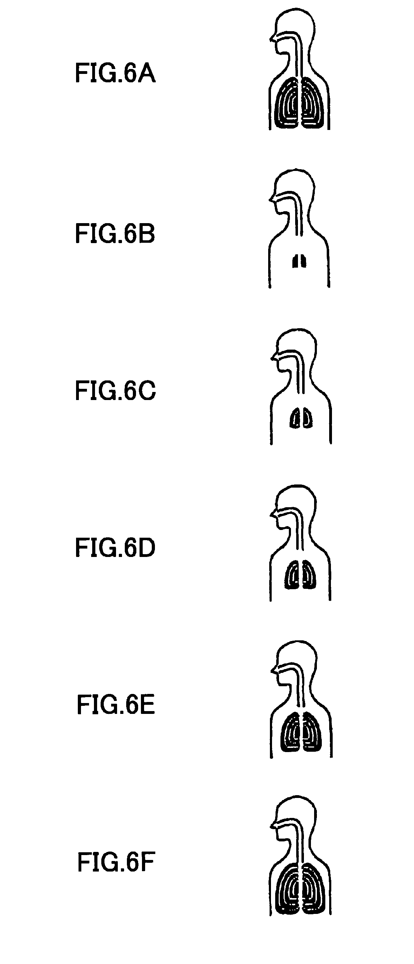

In the blood-pressure monitor in the above embodiment, the seven liquid-crystal-display blocks are arranged in a line, and designed to be turned on stepwise and turned off stepwise so as to display inhalation and exhalation by way of a direction of a change in length of liquid-crystal-display blocks to be turned on. Alternatively, as shown in FIG. 5, a plurality of liquid-crystal-display blocks may be concentrically arranged, and designed to be turned on outward from the innermost block stepwise and turned off inward from the outermost block so as to display inhalation and exhalation by way of a direction of a change in radial size of liquid-crystal-display blocks to be turned on. In yet another embodiment of the blood-pressure monitor of the present invention, a graphic modeled after respiratory organs as shown in FIG. 6A may be used. In this embodiment, inhalation and exhalation is displayed by way of a change in the displayed status of the graphic modeled after respiratory organs, by turning LCD representing the lungs of the respiratory organs on sequentially from the displayed status shown in FIG. 6B to that of FIG. 6F, and by turning LCD off from FIG. 6F to FIG. 6B, respectively.

Further, the control section 4 in FIG. 1 may be provided with audio generation means. In this case, the blood-pressure monitor may be designed to generate a voice "Please inhale air" from the audio output section 10 composed of a speaker or the like when the inhalation timer is set up in Step S10 in FIG. 3 and to generate a voice "Please exhale air" from the audio output section 10 when the exhalation timer is set up in Step S 15. This makes it possible to reliably guide breathing even if a subject shuts his/her eyes to avoid mental tension or is a visually handicapped person, so as to perform a stable blood-pressure measurement.

Furthermore, the control section 4 in FIG. 1 may be provided with current control means for informing a subject about a breathing guidance in an optical manner to change a light intensity of the optical information section 11 composed of a LED or the like in response to breathing. This makes it possible to reliably guide breathing even if a subject has weak sight or is a hearing-impaired person, so as to perform a stable blood-pressure measurement.

In the flowchart in FIG. 3, an inhalation time is set at the same value as that of an exhalation time. Alternatively, an inhalation time may be set at a different value from that of an exhalation time. For example, based on human physiology, an inhalation time may be set at a value greater than that of an exhalation time.

## Claims

1. A blood-pressure monitor comprising:
signal generation means for generating a breathing-guidance signal during a blood-pressure measurement; and
display means for displaying an indication for guiding breathing based on said breathing- guidance signal.

2. The blood-pressure monitor as defined in claim 1, wherein said breathing-guidance signal comprises an exhalation signal and an inhalation signal.

3. The blood-pressure monitor as defined in claim 2, wherein said display means displays inhalation and exhalation by way of a change in the intensity of a bar code.

4. The blood-pressure monitor as defined in claim 2, wherein said display means displays inhalation and exhalation by way of a change in the size of a graphic displayed.

5. The blood-pressure monitor as defined in claim 2, wherein said display means displays inhalation and exhalation by way of a change in the displayed status of a graphic modeled after respiratory organs.

6. The blood-pressure monitor as defined in either one of claims 1 to 5, wherein said signal generation means is operable to continuously generate said breathing-guidance signal during the blood-pressure measurement.
